# EUROPEAN PATENT APPLICATION

(11) **EP 3 984 588 A1**
(43) Date of publication of application: **20.04.2022**
(21) Application number: 21202464.0
(22) Date of filing: 13.10.2021
(51) Int. Cl.: A61M 37/00

(54) **TATTOOING AND PERMANENT MAKE-UP DEVICE**

(30) Priority: 14.10.2020 IT 202000024154
(71) Applicant: Biotek S.r.l., 20129 Milano (MI) (IT)
(72) Inventor: FROIO, Massimo, I-20129 Milano (MI) (IT)
(74) Representative: Balsamo, Andrea

(57) **Abstract**

The present invention relates a device (10) for applying a tattoo or a permanent make-up or a similar skin treatment.The device has a grip portion (11) containing a drive unit, and a disposable needle-holder cartridge (13) which can be coupled to the grip portion (11).The needle-holder cartridge (13) comprises an attachment part (20) for attachment to the grip portion (11), a needle-holder head (28) with a stem (30) which partially protrudes from the attachment part (20), a needle supported by the needle-holder head (28), a tip (25) having a needle-guide nozzle (27).The needle-holder head (28) is arranged in the needle-holder cartridge (13) for a linear movement along the axial direction of the needle-holder cartridge (13), in order to cause the oscillation of the end of the needle in and out of the tip (25) of the needle-holder cartridge (13).The drive unit comprises an electric motor (M) having a drive shaft (15), an actuator shaft (41) which can be coupled to the stem (30) of the needle-holder head (28), a cam mechanism (16,17) operatively connected to the drive shaft (15), for transforming the rotary motion of the drive shaft (15) into a linear motion of the actuator shaft (41). The attachment part (20) of the needle-holder cartridge (13) is provided with electric conducting means (22) adapted to come into contact with the electric terminals (21) of the electric circuit for energizing the electric motor (M) of the device only when the needle-holder cartridge (13) is operatively mounted on the grip portion (11) of the device, in order to establish electric continuity between the electric terminals (21) of the electric circuit for energizing the electric motor (M) of the device. The electric terminals (21) are configured as movable contacts mounted on springs (50) in order to ensure a stable electrical connection between the conducting means (22) and the electric terminals (21) in any adjustment condition of the needle-holder cartridge (13) position relative to the grip portion (11) of the device.

## Description

### Field of the invention

The present invention relates to a device for performing artistic tattooing, aesthetic tattooing, permanent make-up, tricopigmentation and in general pigmentation of any area of the human body.

### Prior Art

As is known, artistic tattooing, aesthetic tattooing, permanent make-up (PMU), tricopigmentation, dermopigmentation and microneedling are all treatments which have the purpose of implanting a pigment with different effects in the human skin. Currently, in order to perform these treatments, conventionally devices provided with a single-use, i.e. disposable, needle-holder cartridge and operated by an electric motor are used.

A lot has been done in order to make these devices safer, more hygienic and easier to use. However, despite the constant improvement of the devices which are nowadays commercially available, there are a number of technical problems negatively affecting the safety, effectiveness, reliability and precision of insertion of the needle in the skin which have not yet been fully resolved. Moreover, the presence on the market of low-cost, poor-quality, devices compromises all the efforts made hitherto to make the aforementioned skin treatments safer.

Among the main technical problems still unresolved in the state of the art the following may be mentioned.

A first problem relates to the fact that, in the known devices, the movement of the needle in the needle-holder cartridge, is not synchronous with movement of the needle drive shaft in the device. This happens because the needle drive shaft and the needle-holder head are not stably in contact with each other such that, whereas the movement of the drive shaft is continuous during operation of the device, the movement of the needle-holder head is instead intermittent.

A second problem concerns the absence in the devices of the prior art of means for ensuring protection against accidental operation of the apparatus when the needle-holder cartridge is not correctly mounted on the device. Without adequate protection means, the known devices may be started by the operator even if the needle-holder cartridge is incorrectly mounted, with the risk of injury both for the operator and for the person undergoing the skin treatment.

A third problem of the known devices consists in the fact that they are not provided with effective means for damping the vibrations generated by the rotation of the electric motor of the device and the transmission which converts the rotary motion of the drive shaft into an alternating motion of the drive shaft of the needle-holder head. The vibrations generated during operation of the device negatively affect the precision of the skin treatment, especially when it is carried out on the eye-lash or in the area around the eyes of the patient. Execution of the treatment in these zones requires great precision and stability of the device and undesirable vibrations may result in the needle coming into contact with the cornea of the eye, causing serious and even permanent injury to the person undergoing treatment. A reduction of the vibrations would be desirable also because it would have a clinical and operational advantage for the operator's hand, the operator having often to work with these devices for many hours during the day and being prone to aches and even hand pathologies due to the vibrations transmitted by the device.

Finally, a fourth problem of the known devices, which for a long time has affected users of these devices, consists in the fact that the needle must be very stable during its alternating movement in and out of the tip of the device, in order to perform skin treatment which is as precise as possible. Therefore, the inner walls of the tip of the device, generally speaking, should be as close as possible to the needle shaft, in order to limit any transverse displacements with respect to the direction of movement. The limitation which prevents this solution from being adopted is that,if the moving needle is too close to the inner walls of the device tip, there is not the space necessary for allowing the liquid pigment to flow out of the reservoir, along the needle shaft, towards the end of the needle itself and therefore into the skin of the patient who is undergoing the skin treatment. Therefore, the smaller the space between the needle shaft and the side walls of the tip of the device, the smaller will be the outflow of the liquid pigment. Conversely, the greater this space, the greater will be the instability of the needle and, therefore, the greater will be the imprecision of the treatment performed. The search for a compromise between these opposing operational requirements has hitherto not provided the hoped-for results, and the problem of instability of the needle has hitherto not been resolved in a satisfactory manner.

### Summary of the invention

The present invention aims to provide a definitive solution to the aforementioned problems of the prior art. According to the invention, this purpose is achieved with a device for applying a tattoo or a permanent make-up, or a similar skin treatment, having a grip portion containing a drive unit, and a disposable needle-holder cartridge, which should be coupled to the grip portion. The needle-holder cartridge comprises an attachment part for attachment to the grip portion, a needle-holder head with a stem which partially protrudes from the attachment part, a needle supported by the needle-holder head, and a tip with a needle guide nozzle. The needle-holder head is arranged in the needle-holder cartridge for a linear movement along the axial direction of the needle-holder cartridge, in order to cause the oscillation of the end of the needle in and out of the tip of the needle-holder cartridge. The drive unit comprises an electric motor having a drive shaft, an actuator shaft which can be coupled to the stem of the needle-holder head, a cam mechanism operatively connected to the actuator shaft, for transforming the rotary motion of the drive shaft into a linear motion of the actuator shaft. The attachment part of the needle-holder cartridge is provided with electric conducting means adapted to come into contact with the electric terminals of the electric circuit for energizing the electric motor of the device only when the needle-holder cartridge is operatively mounted on the grip portion of the device, in order to establish electrical continuity between the electric terminals of the electric circuit for energizing the electric motor of the device. In order to allow adjustment of the movement of the needle out of the tip of the needle-holder cartridge, the device is provided with a system which allows the electrical part of the assembly to remain in contact constantly, recovering by means of the system of guides and springs the space resulting from the movement of the cartridge/needle assembly away from the machine during needle adjustment. According to another feature of the invention, for coupling of the actuator shaft with the stem of the needle-holder head, magnetic coupling means are provided at the end of the actuator shaft and, respectively, at the end of the part of the stem of the needle-holder head which protrudes from the attachment part of the needle-holder cartridge. The magnetic coupling means are adapted to stably, but releasably, connect the actuator shaft to the part of the stem protruding from the attachment part of the needle-holder cartridge, when the needle-holder cartridge is operatively mounted on the grip portion.

According to a feature of the invention, the magnetic coupling means are formed by a first magnet fixed to the end of the actuator shaft which is proximal to the end of the stem part of the needle-holder head which protrudes from the attachment part of the needle-holder cartridge, and by a second magnet or magnetizable metallic element fixed to the end of the stem part of the needle-holder head which protrudes from the attachment part of the needle-holder cartridge. The attraction between the first magnet and the second magnet forms what may be called a "direct coupling" where, for each forwards/backwards movement of the actuator shaft, there is a corresponding similar forwards/backwards movement of the needle-holder head and therefore of the needle itself, the two parts being connected together stably, but releasably owing to the attraction of the magnets, when the needle-holder cartridge is operatively mounted on the grip portion. Advantageously, it is thus possible to avoid the asynchronous movement of the devices known in the state of the art, where the drive shaft only rests against the stem of the needle-holder head.

According to another characteristic feature of the invention, the electric conducting means on the attachment part of the needle-holder cartridge are formed by an annular or semi-annular plate of electrically conductive material arranged on the end of the attachment part of the needle-holder cartridge. Once the needle-holder cartridge is operatively mounted on the grip portion of the device, the plate of electrically conductive material establishes an electrical contact between the terminals of the circuit for energizing the electric motor of the device, allowing energizing of said electric motor when the operator presses the button for switching on the device. It should be pointed out that the electric conducting means may establish the electrical continuity of the electric circuit energizing the electric motor of the device only if the needle-holder cartridge has been operatively mounted on the grip portion of the device. For example, if a screw connection is used, the electrical continuity is established only if the needle-holder cartridge is screwed fully onto the grip portion of the device. Only in this situation is it possible to provide an electric contact and close the circuit which allows the current flow from the power supply to the electric motor, thereby determining whether the device itself is working or not. This measure is intended to ensure a greater degree of safety, preventing the device from being able to be operated in unsuitable circumstances or accidentally, avoiding any injury to the operator and the client.

According to a further characteristic feature of the invention, the attachment part of the needle-holder cartridge which serves for connection to the grip portion is made of a plastic material with optimum vibration-absorbing properties, so as to be able to dampen the vibrations generated by the drive unit. Advantageously, the total or partial absorption of the vibrations generated by the drive unit of the device results in a greater precision with regard to incision in the skin and a greater degree of safety in particular during the permanent make-up treatment of the eye-lashes or the area around the eyes. As is known, the treatment of these parts of the patient requires a greater precision and stability of the needle-holder cartridge since an undesirable vibration may result in the needle coming into contact with the cornea of the eye, causing serious and even permanent injury to the person undergoing treatment. The reduction of the vibrations is also advantageous, in clinical and operational terms, for the operator's hand, the operator often working for many hours during the day and the vibrations transmitted by the device may result in aching and even disorders due to the continuous vibrations affecting the operator's hand. Purely by way of example, the plastic materials which can be used for the attachment part of the needle-holder cartridge may be elastic rubber or ABS with a silicone rubber additive. The needle-holder cartridge body is preferably made of a plastic material more rigid than that of the attachment part. For example, ABS may be used as plastic material for the body of the needle-holder cartridge. The body of the needle-holder cartridge and its attachment part may be produced individually and assembled together or may be comoulded, namely moulded as a single piece made of two different plastic materials. According to another characteristic feature of the invention, the needle-holder cartridge has a pointed cone-shaped end part intended to come into contact with the skin of the person undergoing skin treatment. This part forms a reservoir containing the liquid pigment used in the treatment, as well as a guide for the end part of the needle. The needle is supported and guided by a calibrated cylindrical nozzle which is located at the end of the end part of the needle-holder cartridge which is intended to reduce or eliminate the sideways movements of the needle inside the said tip, increasing the working precision and reducing the risk of tattooing lines being formed outside of the desired zones. At the same time, owing to its particular internal form, the calibrated cylindrical nozzle at the end of the end part ensures a suitable flow of the liquid pigment towards the needle tip and therefore towards the skin of the person undergoing treatment, reducing in fact the technical execution time and the trauma affecting the skin part treated. It should be pointed out that the shorter the insertion time of the needle tip in the skin, the smaller will be the trauma for the person being treated, with a consequent rapid recovery and a reduction in the possibility of contracting infections during bleeding of the treated skin part. The particular internal form of the calibrated cylindrical nozzle at the end of the end part of the needle-holder cartridge is such that there are parts protruding from the inner walls of the guide nozzle which, being in contact with the needle shaft during its movement, ensure the absolute stability thereof, preventing any lateral oscillation thereof, alternating with hollow parts which by means of capillarity allow the liquid pigment to flow towards the tip of the said needle. This particular form of the cylindrical guide nozzle is suitable for every type of needle used in practice and reconciles in an innovative manner the two requirements of lateral stability and passage of the liquid pigment.

According to another characteristic feature of the invention, the device is provided with a conical rubber/silicone membrane inside the needle-holder cartridge which prevents the backflow of liquid pigment and organic pigments towards the body of the device. The membrane is however positioned so as to perform a new function, namely retain the needle inside the needle-holder cartridge when the latter is not operatively mounted on the device body, so as to prevent the tip of needle from coming out and accidentally pricking the operator. The invention, while adopting a conical membrane similar to those used in the known devices of the conventional type, proposes positioning it in an inverted manner with respect to the conventional positioning on the stem of the needle-holder head, so that owing to the elastic properties of the rubber/silicone polymers, it is also able to perform the function of preventing the needle from coming out of the tip of the needle-holder cartridge when the latter is not operatively mounted on the device. By mounting the conical membrane in an overturned manner, it is possible to obtain an elastic effect which prevents the needle from coming out, while allowing the membrane to perform the usual function of retaining the liquid pigment in the event of a backflow towards the device or parts thereof.

### Brief description of the drawings

These and other characteristic features of the present invention will become clearer from the following detailed description of a preferred embodiment of the invention, illustrated purely by way of a non-limiting example in the figures of the attached drawings in which:
Fig. 1 is a front elevation and partially longitudinally sectioned view of the device according to the present invention;
Fig. 2 is an enlarged view of the detail A of Fig. 1;
Fig. 3 is a front elevation and longitudinally sectioned view of the needleholdercartridge associated with the device according to the present invention;
Fig. 4 is a front elevation and longitudinally sectioned view of a variant of the needle-holder cartridge associated with the device according to the present invention;
Fig. 5 is a cross-sectional view of the cylindrical needle guide nozzle in the needle-holder cartridge associated with the device according to the present invention; and
Fig. 6 is a partially sectioned view of a variant of the electric terminals of the circuit for energizing the motor of the device according to the present invention.

### Description of the example of embodiments of the invention

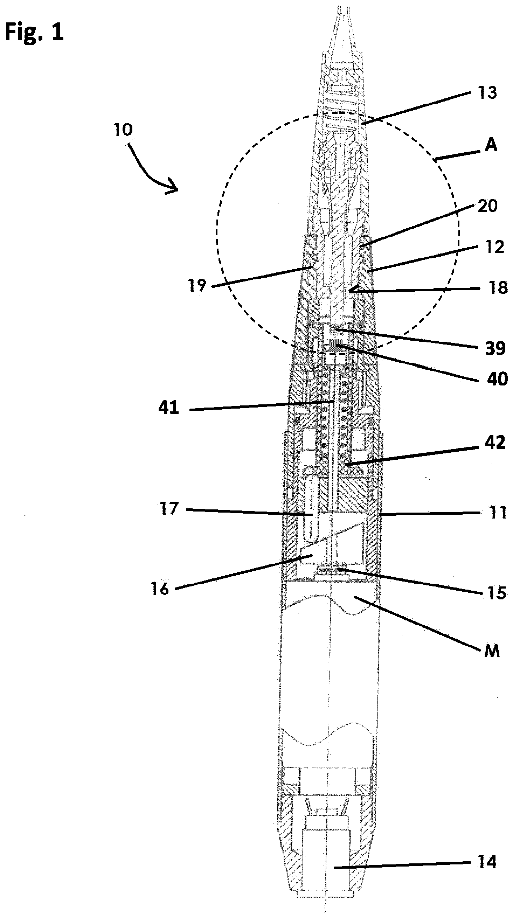

Figure 1 shows a tattooing or permanent make-up (PMU) device according to the present invention, denoted overall by 10. The device 10 comprises, in a manner known per, a body 11, substantially in the form of a writing tool, having a front end part 12, configured to allow fixing of a disposable needle-holder cartridge 13, and a rear connector 14, suitable for connection to a low-voltage electric power source (not shown).

The body 11 of the device houses internally an electric drive motor M, whose drive shaft 15 is connected to a cam 16 of a cam transmission mechanism for transforming the rotary motion of the drive shaft 15 into a reciprocating rectilinear movement of a tappet assembly 17, as typically occurs in tattooing and PMU devices of this kind known in the art.

The front end part 12 of the body 11 has a suitable housing 18 with a threaded part 19 designed to receive a corresponding threaded attachment part 20 of the disposable needle-holder cartridge 13. Obviously, an alternative configuration different from that shown in Figure 1 is also conceivable where the housing with the threaded part is formed on the needle-holder cartridge 13 and the attachment part 20 is formed on the front end part 12 of the body 11. Although below reference will be made to the configuration of Figure 1, it is understood that, for the alternative configuration, identical parts or their variants may be used within the competence of a person skilled in the art.

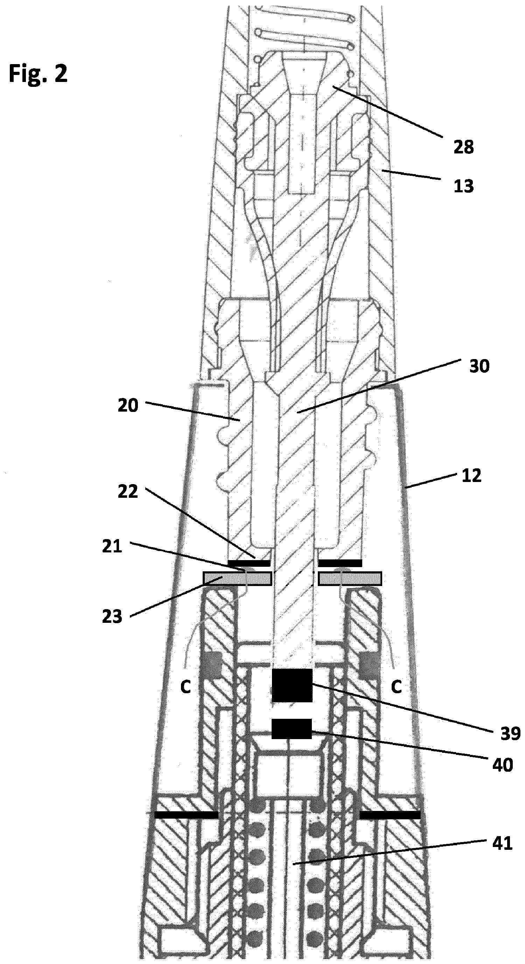

As can be seen more clearly in Figure 2, electric terminals 21 are arranged on the bottom of the housing 20 and, in cooperation with an annular or semi-annular plate 22 of electrically conductive material applied onto the attachment part 20 of the needle-holder cartridge 13, allow an electric contact to be established when the needle-holder cartridge 13 is screwed fully onto the end part 12 of the device 10, in order to excite the electric drive motor M and activate the device. The electric terminals 21 are suitably fixed onto a ring of insulating material 23 and connected to conductors C of the circuit for energizing the electric drive motor M of the device. It should be pointed out that the if attachment part 20 of the needle-holder cartridge 13 is not screwed fully into its housing 18 on the front end part 12, the electric circuit of the motor M is not energized and the device 10 may not be operated.

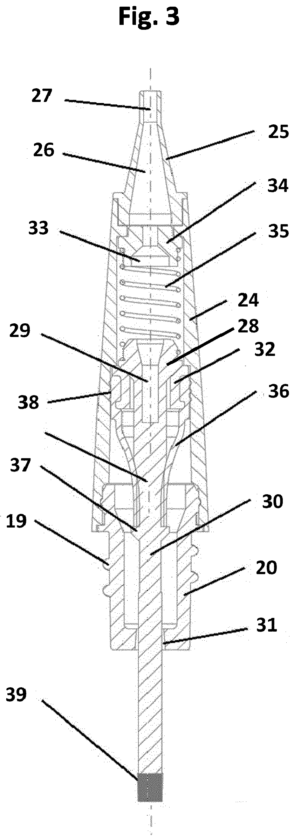

Figure 3 shows in greater detail the needle-holder cartridge 13. This is disposable, namely is intended for use during a single tattooing/PMU session carried out on a single person. The needle-holder cartridge 13 has a hollow central cone-shaped body 24, the rear end of which has, fixed thereto, the attachment part 20 which allows the needle-holder cartridge 13 to be mounted on the front end part 12 of the body 11 of the device 10. The front end of the hollow body 24 of the needle-holder cartridge 13 terminates in a tip 25 which encloses inside it a reservoir 26 for the liquid pigment used in the tattooing/PMU treatment. The tip 25 terminates in a cylindrical guide nozzle 27 for a needle (not shown) of the needle-holder cartridge 13.

A needle-holder head 28 with a receiving cavity 29 for one end of the needle is housed inside the hollow central body 24 of the needle-holder cartridge 13. A stem 30 which passes through the attachment part 20 and protrudes over a certain length from a hole 31 at the rear end of the latter extends from the needle-holder head 28 towards the rear part of the needle-holder cartridge 13. The needle-holder head 28 is seated with a geometric fit inside a support sleeve 32 with a complementary shape which is fixed to the hollow central body 24 of the needle-holder cartridge 13. The end of the needle opposite to that housed inside the needle-holder head 28 is made to pass through a hole 33 in a guide bush 34 for the needle which is located at the front end of the hollow body 24 of the needle-holder cartridge 13.

The needle-holder head 28 is seated inside the support sleeve 32 so as to be displaceable towards the guide bush 34 against the action of a compression spring 35 arranged between the support sleeve 32 and the guide bush 34. The support sleeve 32 is kept stably in position inside the hollow body 24 of the needle-holder cartridge 13 by a flexible sealing element 36 with a conical tubular shape. This flexible sealing element 36 is fitted onto the stem portion 30 between the needle-holder head 28 and a circular protrusion 37 of the stem 30 and is fixed with a geometric and dynamic fit inside an annular cavity 38 between the outer surface of the support sleeve 32 and the inner surface of the hollow central body 24.

At its end in a distal position with respect to the needle-holder head 28, the stem 30 carries a disk-shaped magnet 39, for example a neodymium magnet.

With reference still to Figure 1, it can be seen that a second magnet 40, or alternatively an element made of magnetizable material, is applied to the end of an actuator shaft 41 of the device 10 intended to move the needle-holder head 28 inside the needle-holder cartridge 13. When the needle-holder cartridge 13 is operatively mounted on the body 11 of the device 10, the magnets 39 and 40 are attracted together, forming a so-called "direct coupling" connection, namely an operatively direct and stable, but releasable connection between the stem 30 and the actuator shaft 41.

The actuator shaft 41 is actuated indirectly, via a spring thrust element 42, by the tappet system 17 which, in turn, receives the movement from the cam 16 of the cam transmission mechanism of the device 10.

It should be noted that the so-called "direct coupling" connection between stem 30 of the needle-holder head 28 and the actuator shaft 41 is different from the trend in the state of the art, where the aforementioned elements move by means of a thrust mechanism, but not do remain stably joined together during their movement.

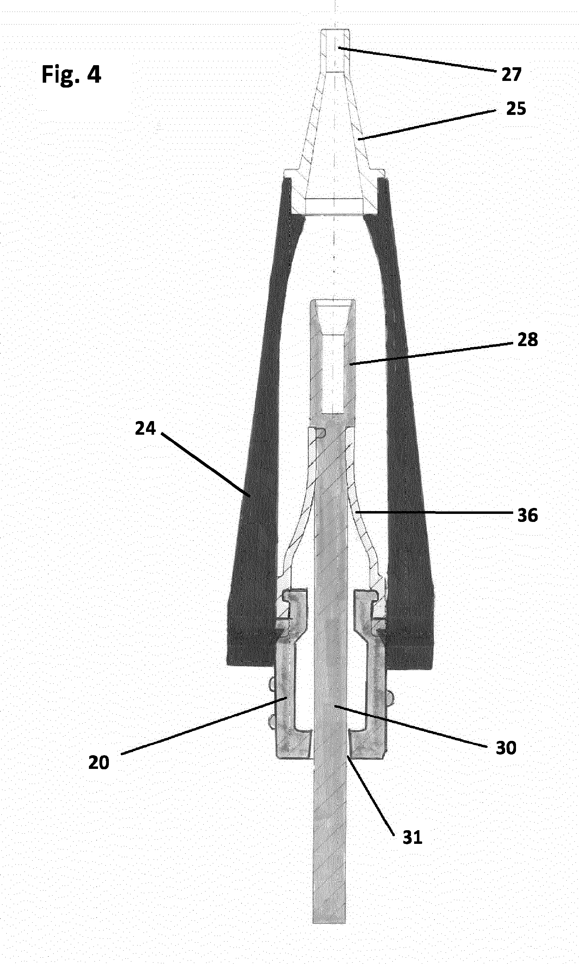

The sealing element 36 is formed by a membrane made of suitable material, for example rubber or silicone, so as to form a hermetic seal and prevent the liquid pigment, together with any body liquids which may flow from the tip 25 as a result of the alternating movement of the needle, from reaching the stem 30 and, consequently, the actuator shaft 41 and the thrust element 42. The sealing element 36 has a tapered form and may be mounted in the configuration shown in Figure 3, or in the configuration shown in Figure 4, i.e. in the exactly opposite manner, and therefore with the narrow part directed towards the tip 25 of the needle-holder cartridge 13.

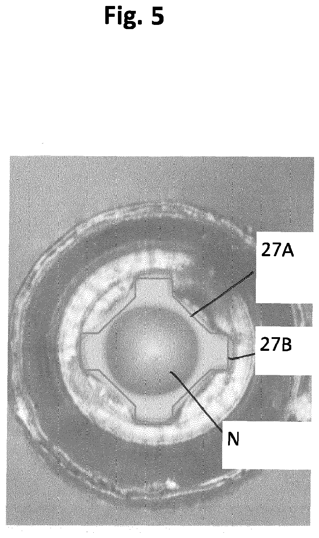

In the case of the variant shown in Figure 4, it must be pointed out that the inherent elasticity of the silicone or rubber membrane which forms the sealing element 36 prevents the needle from coming out of the needle-holder cartridge 13 when the latter is detached from the front end part 12 of the body 11 of the device 10. This is advantageous because the sealing element 36 in this case not only prevents the backflow of the liquid pigment, but also ensures retention of the needle inside the needle-holder cartridge body 13, preventing the operator from being accidently pricked. Moreover, it is pointed out that in the case of the variant shown in Figure 4, it would not be necessary to use the recall spring 35, because the recall action exerted by the same elastic membrane which forms the sealing element 36 may be sufficient. The cylindrical needle guide nozzle, denoted by 27 in Figure 3, is configured internally so as to allow a forwards and backwards movement of the needle without lateral oscillations, allowing however the liquid pigment to pass freely from the reservoir of the needle-holder cartridge 13 to the needle and the skin of the person being treated. The details of the internal form of the cylindrical guide nozzle 27 are shown in Figure 5. Here it is possible to note projecting zones 27a which support and come into contact with the needle N during its movement and hollow zones 27B intended to allow the liquid pigment to pass towards the end of the said needle and the skin of the person being treated.In the example shown in Figure 5, only a single needle is present, but the characteristics of the guide nozzle 27 may be replicated also in the case of tattooing/PMU devices which use several needles. The configuration of the guide nozzle 27 allows a more stable movement of the needle, resulting in a much more precise tattooing/PMU action and the creation of a more intense colour. Since the needle acts on the skin of the person being treated for a very short period of time, the trauma is reduced to a minimum and healing is more rapid.

With reference again to Figure 2, it shows more clearly the magnet 39 which is fitted to the end of the stem 30 of the needle-holder head 28 and the magnet 40 which is fitted onto the actuator shaft 41. The two magnets attract each other with a strong force, creating the aforementioned "direct coupling" connection between the stem 30 of the needle-holder head 28 and the actuator shaft 41, which results in a synchronous movement of said parts, i.e. for each movement of the actuator shaft 41 imparted by the cam transmission mechanism there is a corresponding similar movement of the needle-holder head 28 and therefore of the needle inside the needle-holder cartridge 13. Obviously, the "direct coupling" connection may be provided by means of two magnets or by means of a single magnet and a metal part which may be subject to the force of attraction of the magnet.

Figure 2 also shows the end part of the needle-holder cartridge 13 inside which the annular or semi-annular conducting plate 22, intended to ensure electrical continuity with the electric terminals 21, has been inserted by means of pressing or gluing. The purpose of the conducting plate 22 is to close an electric circuit which allows an operator to start the device 10, by means of a special control and command console (not shown) or by means of any power supply unit which converts the mains voltage from 230/110 volts to 3-24 volts. If there is no electrical contact between the conducting plate 22 and the electric terminals 21, the electric circuit of the device is not closed and the device 10 may not be started. When the conducting plate 22 of the needle-holder cartridge 13 comes into contact with the electric terminals 21, the circuit is closed and switching-on and operation of the device 10 is enabled. The electric terminals 21 also have, connected thereto, wire-like, strip-like or similarly shaped conducting elements, which transmit the electric signal to the electric motor M of the device.

Closing of the electric circuit could be performed also by means of closing of a contact on the motor, using for example a conducting part on the body 11 of the device, or by means of a special electronic control unit arranged between the cables and the electric motor M. In this case, the electric terminals 21 would be connected to an operational amplifier and would function as proximity sensors able to detect variations in the capacitance caused by the approaching movement of the conducting plate 22 located on the attachment part 20 of the needle-holder cartridge 13. The movement of the conducting plate 22 towards the electric terminals 21 would also cause switching of an operational amplifier, enabling the closure of the electric contact for activating the motor M. Alternatively, the closing of the electric circuit could also be achieved by means of conducting parts on the inner or outer walls of the body 11 of the device.

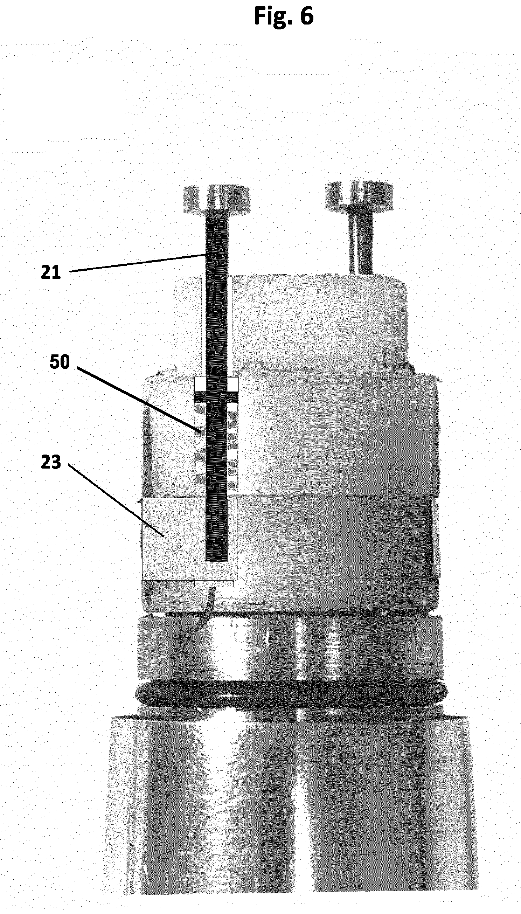

In the device 10 shown in Figure 1, the position of the front end part 12 is adjustable with respect to the body 11 of the device by means of a threaded adjustment system in the front part of the body 11, so as to be able to adjust the amount by which the needle protrudes from the needle-holder cartridge 13. In this case, in order to keep the electrical connection between the conducting plate 22 and the electric terminals 21 constant, it may be advantageous to form these terminals as movable contacts mounted on springs 50, as shown in Figure 6. Owing to the fact that the electric terminals are mounted on springs 50, it is possible to ensure the electrical contact between the electric terminals 21 and the conducting plate 22 in any adjustment condition of the needle-holder cartridge 13.

Although the invention has been described with reference to a preferred example of embodiment shown in the drawings, it is evident that the invention is not limited to the details described and a person skilled in the art may make multiple modifications and variations thereto which may be regarded as being included within the scope of the attached claims.

## Claims

1. Device (10) for applying a tattoo or a permanent make-up, or a similar skin treatment, comprising a grip portion (11) containing a drive unit, and a disposable needle-holder cartridge (13), which can be coupled to said grip portion (11), said needle-holder cartridge (13) comprising an attachment part (20) for attachment to said grip portion (11), a needle-holder head (28) with a stem (30) which partially protrudes from said attachment part (20), a needle supported by said needle-holder head (28), and a tip (25) with a needle guide nozzle (27), said needle-holder head (28) being arranged in said needle-holder cartridge (13) for a linear movement along the axial direction of the needle-holder cartridge (13), in order to cause oscillation of the end of the needle in and out of said tip (25) of the needle-holder cartridge (13), said drive unit comprising an electric motor (M) having a drive shaft (15), an actuator shaft (41) which can be coupled to the stem (30) of needle-holder head (28), a cam mechanism (16, 17) operatively connected to said drive shaft (15), for transforming the rotary motion of the drive shaft (15) into a linear motion of said actuator shaft (41), **characterized in that** said attachment part (20) of the needle-holder cartridge (28) is equipped with electric conducting means (22) adapted to come into contact with electric terminals (21) of the electric circuit for energizing said electric motor (M) of the device only when the needle-holder cartridge (13) is operatively mounted on said grip portion (11) of the device, in order to establish an electric continuity between said electric terminals (21) of the electric circuit for energizing said electric motor (M) of the device, said electric terminals (21) are configured as movable contacts mounted on springs in order to ensure a stable electrical connection between the conducting means (22) and the electric terminals (21) in any adjustment condition of the needle-holder cartridge (13) position relative to the grip portion (11) of the device.

2. Device according to claim 1, **characterised in that** for coupling of said actuator shaft (41) with the stem (30) of the needle-holder head (28), magnetic coupling means (39, 40) are provided at the end of said actuator shaft (41) and, respectively, at the end of said part of the stem (30) of the needle-holder head (28) which protrudes from said attachment part (20) of the needle-holder cartridge (13), said magnetic coupling means (39, 40) being adapted to stably, but releasably, connect said actuator shaft (41) to the stem part (30) protruding from said attachment part (20) of the needle-holder cartridge (13) when said needle-holder cartridge (13) is operatively mounted on said grip portion (11), and **in that**

3. Device according to claim 2, **characterized in that** the magnetic coupling means (39, 40) are formed by a first magnet (40) fixed to the end of the actuator shaft (41) which is proximal to the end of the stem part (30) of the needle-holder head (28) which protrudes from the attachment part (20) of the needle-holder cartridge (13), and a second magnet or magnetizable element (39) fixed to said end of the stem part (30) of the needle-holder head (28) which protrudes from the attachment part (20) of the needle-holder cartridge (13), and **in that** said proximal ends of the actuator shaft (41) and of the stem (30) of the needle-holder head (28) are connected stably, but releasably, to each other, due to the attraction between said first magnet (40) and said second magnet or magnetisable element (39), when the needle-holder cartridge (13) is operatively mounted on the grip portion (11) of the device.

4. Device according to claim 1, **characterized in that** the electrical conducting means (22) on the attachment part (20) of the needle-holder cartridge (13) are formed by an annular or semi-annular plate of electrically conductive material arranged on an end portion of the attachment part (20) of the needle-holder cartridge (13).

5. Device according to claim 1, **characterized in that** the attachment part (20) of the needle-holder cartridge (13) which serves for the connection to the grip portion (11) of the device is made of a plastic material with optimum vibration-absorbing properties, so as to be able to dampen the vibrations generated by the drive unit.

6. Device according to claim 1, **characterized in that** the needle-holder cartridge (13) has an end part which forms a tip (25) intended to come into contact with the skin of the subject undergoing a skin treatment, said end part (25) forming a reservoir (26) containing the liquid pigment used in the treatment together with the end part of the needle shaft, and comprising a needle guide nozzle (27) which supports and guides the needle shaft during its linear movement along the axial direction of the needle-holder cartridge (13), said needle guide nozzle (27) having parts (27A) which protrude from its inner walls and are in contact with the needle shaft during its movement to prevent lateral oscillation thereof, said protruding parts (27A) being arranged alternating with hollow portions (27B), which by means of capillarity allow the liquid pigment to flow out from said tip (25).

7. Device according to claim 1, **characterized in that** the needle-holder cartridge (13) is internally provided with a conical membrane (36) of flexible material which prevents the backflow of liquid pigment and organic liquids towards the grip portion (11) of the device, said membrane (36) being positioned so as to resiliently retain the needle inside the needle-holder cartridge (13) when the latter is not operatively mounted on the grip portion (11) of the device.
